# EUROPEAN PATENT APPLICATION

(11) **EP 2 466 311 A1**
(43) Date of publication of application: **20.06.2012**
(21) Application number: 10195621.7
(22) Date of filing: 17.12.2010
(51) Int. Cl.: G01N 33/68

(54) **sFlt1 in patients with ischemic stroke**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Zdunek, Dietmar, 82327 Tutzing (DE); Horsch, Andrea, 68259 Mannheim (DE)
(74) Representative: Dick, Alexander

(57) **Abstract**

The present invention relates to a method for diagnosing cardiac ischemia associated with an ischemic stroke. Said method is based on determining the amount of sFlt1 in a sample from said patient, and comparing the amount to a reference amount. Moreover, the present invention envisages a method for monitoring cardiac ischemia in a patient with ischemic stroke and a method of identifying a subject being susceptible to a further therapy for lowering the blood pressure. Said methods are based on determining the amount of sFlt1 in a first and second sample from said patient, and comparing the amount in the first sample to the amount in the second sample. Further encompassed by the present invention are kits and devices for carrying out the said methods.

## Description

The present invention relates to a method for diagnosing cardiac ischemia associated with ischemic stroke. Said method is based on determining the amount of sFlt1 in a sample from said patient, and comparing the amount to a reference amount. Moreover, the present invention envisages a method for monitoring cardiac ischemia in a patient with ischemic stroke and a method of identifying a subject being susceptible to a further therapy for lowering the blood pressure. Said methods are based on determining the amount of sFlt1 in a first and second sample from said patient, and comparing the amount in the first sample to the amount in the second sample. Further encompassed by the present invention are kits and devices for carrying out the said methods.

Stroke ranks after ischemic heart disease second as a cause of lost disability-adjusted life-years in high-income countries and as a cause of death worldwide. Acute stroke is caused by a cascade of events from energy depletion to cell death after arterial occlusion. A central core of very low perfusion is surrounded by an area of dysfunction caused by metabolic and ionic disturbances, but in which structural integrity is preserved. In case reperfusion of the ischemic area is not achieved, irreversible damage will occur (Dirnagel U. et al, Trends in Neuroscience 1999: 22: 391-7).

Clinically acute ischemic stroke is typically characterized by the sudden onset of a focal neurologic deficit, common deficits include dysphasia, dysarthria, hemianopia, weakness, ataxia, sensory loss, and neglect. Symptoms and signs are unilateral and consciousness is in general normal or slightly impaired. Ischemic stroke may be mimicked by migraine, postictal paresis, hypoglycaemia, conversion disorder, subdural hematoma or brain tumors. In patients with transitory ischemic stroke, symptoms may be temporary and may have disappeared as the patient is seen by the physician which creates diagnostic difficulties for the attending physician (van der Worp B and van Gijn J. et al, NEJM 2007: 357: 572 - 578).

Several diagnostic tests shall be carried out routinely in patients with ischemic stroke to identify systemic conditions that may cause stroke. It has been described that myocardial ischemia such as unstable angina or myocardial infarction may be the trigger for ischemic stroke (see Adams H.P. et al Stroke 2007: 38: 1655 - 711. This observation led to the recommendation of ECG monitoring during the first 24 hours of stroke, since subjects both suffering from cardiac ischemia and from ischemic stroke are at significantly increased risk of mortality.

Moreover, an ischemic stroke may also be the cause for ischemia itself. E.g. it has been described that stroke-induced cardiac ischemia significantly contributes to poststroke cardiac morbidity and mortality. It is, therefore, particularly important to assess cardiac ischemia in subjects after the onset of stroke symptoms.

Several factors are considered to cause cardiac ischemia in stroke patients. One of these reasons considered to cause cardiac ischemic is aggressive antihypertensive treatment which aims to lower the blood pressure in patients with stroke.

Elevated blood pressure is often detected in the first hours after stroke. E.g. Robinson et al. (The predictive role of 24-hour compared to causal blood pressure levels on outcome following acute stroke. Cerebrovase Dis. 1997; 7: 264-272) describes that blood pressure of larger than 160 mm Hg are detected in more 60% of patients with acute stroke. High blood pressure is an indicator for poor outcome. Therefore, a therapy that aims to lower the blood pressure is frequently initiated after the diagnosis of stroke. Theoretical reasons for lowering blood pressure include reducing the formation of brain edema, lessening the risk of hemorrhagic transformation of the infarction, preventing further vascular damage, and forestalling early recurrent stroke. (see Adams et al., loc. cit.)

There is significant uncertainty about lowering blood pressure in stroke patients. Aggressive treatment of blood pressure may lead to neurological worsening by reducing perfusion pressure to ischemic areas of the brain and may even result in acute cardiovascular events.

Another important aspect of stroke is interaction with preexisting cardiac disease. In a large study in stroke patient with pre-existent cardiac disease ECG abnormalities were frequent and were found in 55.3 % of patients (Christensen H. et al J Neurolog Sciece 2005: 234: 99 - 103). Even more importantly ST segment changes resulting from the stroke were also found (Christensen H et al.), also changes in heart rate could be found (with a significant decline over time). This is further evidence that ischemic stroke has impact on cardiac function. This is further supported by the observation that heart failure patients with low output may suffer from increased vulnerability of the brain by hypoperfusion and a decreased global (cerebral) blood flow. This fact and the presence of altered cerebrovascular reactivity makes these patients also vulnerable to hypotension (Pullicino P.M. et al, Stroke 2009: 40: 3706 - 3710). The need to retain systolic blood pressure in stroke is however not consistent with heart failure therapy recommendations to maintain systolic blood pressure at the lowest tolerable level which is achieved with ACE inhibitors, beta blockers and diuretics (Pullicino P.M. et al). Thus there is a therapeutic dilemma with currently no diagnostic methods available. A further aspect of hypoperfusion comes from inadequate perfusion of other parts of the body (e.g. the heart in case of severe coronary artery disease with evidence from ST segment depression after stroke, Christensen H et al) or the lower and upper limbs of the body resulting in ischemia which may remain unnoticed in case of stroke (among other factors the patients inability to speak).

Thus, recognition of ischemia, preferably cardiac ischemia secondary to stroke appears an important aspect in its treatment and has therapeutic consequences such as discontinuation of drugs that lower blood pressure or improvement of therapy of hypertension in case of high blood pressure.

Therefore, it is particularly important to monitor ischemia (in particular cardiac ischemia) and therapies for lowering the blood pressure in patients with stroke.

Soluble Flt-1 (sFlt-1, soluble fins-like tyrosine kinase-1, frequently also referred to as VEGF receptor-1) is a splice variant of VEGF receptor 1 which is produced by a variety of tissues. sFlt-1 binds the proangiogenic factors vascular endothelial growth factor (VEGF) and placental growth factor (PlGF). sFlt1 is used - in combination with PlGF - as a marker for discriminating between uncomplicated pregnancy and pregnancy with preeclampsia (Troisi et al. (2008) Am J Obstet Gynecol 199(6)).

US2007/218498 proposes sFlt1 as a marker for several cardiovascular and cerebral conditions, inter alia, for myocardial ischemia, ischemic stroke, intracerebral hemorrhage, subarachnoid hemorrhage, transient ischemic attack. Thus, US2007/218498 discloses sFlt1 as a general marker for cardiac and cerebral ischemia.

As mentioned above, it is desirable to diagnose whether cardiac ischemia is the cause for ischemic stroke. Moreover, it is important to monitor ischemia, in particular cardiac ischemia, after the onset of symptoms of ischemic stroke. Also, it is desirable to identify subjects suffering from ischemic stroke which are susceptible to a further therapy for lowering the blood pressure.

The technical problem underlying the present invention can be seen as the provision of means and methods which comply with the aforementioned needs. The technical problem is solved by the embodiments characterized in the claims and herein below.

Accordingly, the present invention relates to a method for diagnosing whether cardiac ischemia is associated with, preferably the cause, of an ischemic stroke in a subject suffering from an ischemic stroke, said method comprising:
a) determining the amount of sFlt1 (soluble fins-like tyrosine kinase-1) in a sample from said subject, and
b) comparing the amount of sFlt1 in said sample to a reference amount, whereby it is diagnosed whether cardiac ischemia is associated with said ischemic stroke (preferably, the cause of said ischemic stroke).

Preferably, it is diagnosed whether cardiac ischemia is associated with, preferably the cause of said ischemic stroke by carrying out the further step of c) diagnosing whether cardiac ischemia is associated with said ischemic stroke based on the result of the comparison carried out in step b). Preferably, it is diagnosed in step c) whether cardiac ischemia is the cause of ischemic stroke.

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein, preferably, means assessing whether cardiac ischemia is associated with ischemic stroke in a subject suffering from an ischemic stroke. More preferably, the term means assessing whether cardiac ischemia is the cause of ischemic stroke in a subject suffering from an ischemic stroke. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "associated with" as used herein, preferably, refers to a temporal and/or causal relationship between cardiac ischemia and ischemic stroke. The person skilled in the art understands what is meant if cardiac ischemia is considered to be associated with an ischemic stroke. Particularly, a cardiac ischemia shall be considered to be associated with an ischemic stroke, if it is present within 15 minutes, 1, 2, 3, or 4 hours before the onset of symptoms of stroke and/or within 15 minutes, 1, 2, 3, 4, 5, 6, 7, 8, 9, 10, 11, 12, 15, 18, 21, or 24 hours after the onset of symptoms of stroke. Alternatively or additionally, cardiac ischemia shall be considered to be associated with an ischemic stroke if the ischemic stroke is causally related said cardiac ischemia, whether directly or indirectly. Indication for such causal connection is e.g. a close time-relationship between cardiac ischemia and ischemic stroke (see immediately above). With the expression "cardiac ischemia associated with ischemic stroke" it is, preferably, meant that the cardiac ischemia is the cause of the ischemic stroke. Thus, by diagnosing cardiac ischemia associated with ischemic stroke, it is, preferably, possible to diagnose whether cardiac ischemia is the cause of ischemic stroke. More preferably, it shall be possible to diagnose whether cardiac ischemia is the precipitating cause of ischemic stroke.

The term "subject" as used herein relates to animals, preferably mammals, and, more preferably, humans. The subject according to the present invention shall suffer from ischemic stroke. More preferably, said subject does or not suffer from acute infections. Also preferably, the subject in the context with the aforementioned method shall have normal kidney function. Furthermore, the subject is, preferably, a subject presenting to an emergency unit.

The term "ischemic stroke" (herein also referred to as "stroke") is well understood by the skilled person (see e.g. Adams et al., Guidelines for the Early Management of Adults With Ischemic Stroke, A Guideline From the American Heart Association/ American Stroke Association Stroke Council, Clinical Cardiology Council, Cardiovascular Radiology and Intervention Council, and the Atherosclerotic Peripheral Vascular Disease and Quality of Care Outcomes in Research Interdisciplinary Working Groups in Stroke. 2007;38:1655 which is herewith incorporated by reference with respect to its entire disclosure content). As used herein, the term, preferably, refers to cerebral ischemic stroke. Moreover, it preferably refers to a stroke which is caused by reduced blood flow to the brain or parts thereof which leads to a reduced delivery (undersupply) of oxygen to brain cells. Preferably, the ischemic stroke is characterized by tissue anemia caused by obstruction of the inflow of arterial blood. Preferably, the ischemic stroke leads to irreversible tissue damage due to brain cell death. Ischemic stroke may be caused by atherothrombosis or embolism of a major cerebral artery, by coagulation disorders or nonatheromatous vascular disease, or by cardiac ischemia which leads to a reduced overall blood flow. The ischemic stroke is preferably selected from the group consisting of atherothrombotic stroke, cardioembolic stroke and lacunar stroke.Determination of the type stroke is known to the person skilled in the art and includes different imaging techniques such as echocardiography, electrocardiogram and doppler ultrasound. Preferably, the ischemic stroke is an acute ischemic stroke.

The term "ischemic stroke" does, preferably, not include hemorrhagic stroke.

Whether a subject suffers from stroke, in particular from ischemic stroke can be determined by well known methods. Moreover, symptoms of stroke are well known in the art and e.g. described in Adams et al. (loc. cit.). E.g., stroke symptoms include sudden numbness or weakness of face, arm or leg, especially on one side of the body, sudden confusion, trouble speaking or understanding, sudden trouble seeing in one or both eyes, and sudden trouble walking, dizziness, loss of balance or coordination.

In the context of the method of the present invention, the ischemic stroke may differ in its severity. For example, the stroke may be a major ischemic stroke or a minor stroke. Furthermore, the term "ischemic stroke" preferably also included transient ischemic attacks (TIAs). A TIA, preferably, is a brief episode of neurological dysfunction caused by insufficient cerebral blood supply to the brain that, preferably, lasts less than 2 hours, less than an hour, or less than 30 minutes without evidence of infarction.

The term "cardiac ischemia" as used herein, preferably, refers to a condition characterized by a reduced blood flow to the heart leading to a reduced delivery of oxygen delivery (undersupply) to cells comprised by the cardiac tissue. Preferably, cardiac tissue is affected by ischemia, if the amount of oxygen that is supplied to said tissue is not sufficient in order to cover the need of the cells comprised by said tissue, and, thus, to meet the rate of mitochondrial oxidation in said cells. As a consequence of said reduced delivery myocardial necrosis and/ myocardial hibernation may occur. It is, particularly, contemplated that the term "cardiac ischemia" refers cardiac ischemia associated with an acute coronary syndrome (ACS). ACS includes unstable angina pectoris (UAP) or myocardial infarction (MI). MI can be an ST-elevated MI (STEMI) or a non-ST-elevated MI (NSTEMI). Thus, the determination of sFlt1 in a subject and the comparison of said amount to a reference amount as described herein, preferably, allows for assessing whether an acute coronary syndrome associated with an ischemic stroke (preferably, is the cause of said stroke). Thus, in the context with the aforementioned method "cardiac ischemia" may mean UAP, STEMI or NSTEMI. Particularly, the term "cardiac ischemia" refers to cardiac ischemia associated with unstable angina pectoris (UAP).

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Preferably, the sample in the context with the aforementioned method shall be obtained after the onset of symptoms of said ischemic stroke. Preferably, the sample shall be obtained not later than 12 or 24 hours after the onset of said symptoms. More preferably, the sample is, preferably, obtained not more than one, two, three, four, five, six or nine hours after the onset of symptoms of ischemic stroke. It is also preferred that the sample is obtained not earlier than 15 minutes, but not later than nine hours, more preferably, not earlier than one hour but not later that six hours, even more preferably, not early than one hour, but not later than to five hours, and most preferably, not earlier than two hours but not later than three hours after the onset of symptoms of ischemic stroke. It is particularly, envisaged that the sample is obtained at or after the first physical examination carried out after the onset of stroke symptoms such as when the subjects presents to an emergency physician or at the emergency room.

The term "soluble Flt-1" or "sFlt-1" (abbreviation for Soluble fins-like tyrosine kinase-1) as used herein refers to polypeptide which is a soluble form of the VEGF receptor Flt1. It was identified in conditioned culture medium of human umbilical vein endothelial cells. The endogenous soluble Fltl (sFltl) receptor is chromatographically and immunologically similar to recombinant human sFlt1 and binds [125I] VEGF with a comparable high affinity. Human sFlt1 is shown to form a VEGF-stabilized complex with the extracellular domain of KDR/Flk-1 in vitro. Preferably, sFlt1 refers to human sFlt1 as described in Kendall 1996, Biochem Biophs Res Commun 226(2): 324-328 (for amino acid sequences, see, e.g., also P17948, GI: 125361 for human and BAA24499.1, GI: 2809071 for mouse sFlt-1). The term also encompasses variants of the aforementioned human sFlt-1 polypeptides. Such variants have at least the same essential biological and immunological properties as the aforementioned sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide, preferably over the entire length of the human sFlt-1, respectively. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981 ), by the homology alignment algorithm of Needleman and Wunsch J. Mol. Biol. 48:443 (1970 ), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments or subunits of the specific sFlt-1 polypeptides or the aforementioned types of variants as long as these fragments have the essential immunological and biological properties as referred to above. Such fragments may be, e.g., degradation products of the sFlt-1 polypeptides. Further included are variants which differ due to posttranslational modifications such as phosphorylation or myristylation.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemilumidescence sandwich immunoassays (ECLIA), dissociation-enhanced lanthanide fluoro immune assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether cardiac ischemia is associated with ischemic stroke. By assessing, whether cardiac ischemia is associated with ischemic stroke, it is, preferably, possible to assess whether cardiac ischemia is the cause for ischemic stroke.

The term "reference amount" as used herein refers to an amount which allows for allocation of a subject into either the group of subjects in which cardiac ischemia is associated with (preferably, is the cause for) said ischemic stroke or in which cardiac ischemia is not associated with (preferably, is not the cause for) said ischemic stroke. Such a reference amount can be a threshold amount which separates these groups from each other. Accordingly, the reference amount for a the biomarker sFlt1 shall be an amount which allows for allocation of a subject into a group of subjects in which cardiac ischemia is associated with (preferably, is the cause for) said ischemic stroke or into a group of subjects cardiac ischemia is not associated with (preferably is not the cause for) for said ischemic stroke. A suitable threshold amount separating the two groups can be calculated without further ado by the statistical tests referred to herein elsewhere based on amounts of a sFlt1 from either a subject or group of subjects suffering from ischemic stroke known to have cardiac ischemia associated with (preferably known to have cardiac ischemia as a cause for) ischemic stroke or a subject or group of subjects suffering from ischemic stroke known not to have cardiac ischemia associated with (preferably known not to have cardiac ischemia as a cause for) for ischemic stroke. Preferred referenced amounts which can be derived from the aforementioned subjects or group of subjects are indicated elsewhere herein. Preferably, the reference amount is derived from a subject or a group of subjects known to suffer from an ischemic stroke, wherein cardiac ischemia is associated with ischemic stroke (preferably, wherein cardiac ischemia is the cause for said stroke). In this case, an essentially identical amount or an increased amount of sFlt1 in the test sample as compared to reference amount is, preferably, indicative for cardiac ischemia is associated with ischemic stroke. More preferably, is indicative for cardiac ischemia as a cause for said ischemic stroke.

Also preferably, the reference amount may be derived from a subject or a group of subjects known to suffer from an ischemic stroke, wherein cardiac ischemia is not associated with ischemic stroke (preferably, is not the cause for said ischemic stroke). In this case, an essentially identical amount or a decreased amount of sFlt1 in the test sample as compared to reference amount indicated that cardiac ischemia is not associated with ischemic stroke. More preferably, it indicates that cardiac ischemia is not the cause for said ischemic stroke.

The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Preferred reference amounts are indicated herein below:
A preferred reference amount indicating that cardiac ischemia associated with ischemic stroke (preferably, is the cause for ischemic stroke) is an amount of sFlt1 of about 100 pg/ml to about 250 pg/ml and, more preferably, 100 to 150 pg/ml, about 100 to about 130 pg/ml, even more preferably of about 90 to 110, or of about 100 to 110 pg/ml. Even more preferably, the reference amount of about 100, 110, 120, 130 or 150 pg/ml. A test amount being essentially identical or increased shall be indicative for cardiac ischemia associated with (preferably, as cause of) ischemic stroke while a decreased amount shall be an indicator that cardiac ischemia is not associated with ischemic stroke (preferably, is not the cause for ischemic stroke).

The term "about" in the context of the present invention means +/- 20%, +/- 10%, +/- 5%, +/- 2 % or +/- 1% from the said values. This also takes into account usual deviations caused by measurement techniques, statistics and the like.

Reference amounts can, in principle, be calculated for a cohort of subjects as specified above based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/- specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention can be, preferably, a threshold or cut off amount and can be generated, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject in which cardiac ischemia is the cause for ischemic stroke (i.e. a rule out) whereas an optimal specificity is envisaged for a subject in which cardiac ischemia is the cause for ischemic stroke (i.e. a rule in).

Also preferably, the amounts of sFlt1 in the accompanying Examples in subjects in which cardiac ischemia was associated with ischemic stroke may be also used as a basis for establishing thresholds.

Interestingly, is has been found in the context of the studies underlying the present invention that sFlt1 is a reliable marker for diagnosing cardiac ischemia associated with an ischemic stroke in a subject suffering from an ischemic stroke. Particularly, increased amounts of sFlt1 in sample from a subject suffering from ischemic stroke indicate that cardiac ischemia associated with ischemic stroke, whereas decreased amounts in a sample indicate that cardiac ischemia is associated for ischemic stroke. This observation was surprising since sFlt1 was thought to be a general marker for ischemic cerebral and cardiac conditions (see US2007/218498). It now, however, has been found that sFlT1 is generally not increased in ischemic stroke at presentation to the physician (see Examples). Interestingly, however, three patients with ischemic stroke had significantly increased sFlT1 levels (which levels even exceeded the 95 percentile of coronary artery disease, see Examples). These increased sFlt1 level indicate that cardiac ischemia is the cause for ischemic stroke. Interestingly, cardiac troponin T which is a marker for myocardial necrosis was not increased in two of the three patients with significantly increased sFlt1 levels. Thus, the determination of sFlt1 allows the diagnosis of cardiac ischemia even in patients in which cardiac ischemia does not result in myocardial necrosis (e.g. in patient with unstable angina). The lack of sFlT1 increase shortly after the stroke offers the opportunity to identify cardiac ischemia associated with (preferably as cause of) stroke in patients presenting with stroke. The identification of the of cardiac ischemia, particularly the cause of ischemia, is important for the further therapeutic or diagnostic steps as envisaged. Thanks to the present invention it is now possible to direct diagnostic or therapeutic approaches towards more intense cardiac diagnostic or therapeutic procedures.

Thus, in a preferred embodiment of the method of the present invention, said method further comprises recommending a therapy for said subject. A therapy that can be recommended in subject who suffered from an ischemic stroke associated with (preferably, cause by) cardiac ischemia, is, e.g. percutaneous coronary intervention (PCI).

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention. If the method of the present invention shows that the ischemic stroke is associated with cardiac ischemia, then the subject preferably shall be subjected to a cardiac follow up, preferably by providing an electrocardiogram or by determining the amount of cardiac Troponin T in a sample from said patient. In case of ECG changes or troponin elevations, PCI should be considered depending on the severity of stroke.

It is to be understood that the definitions and explanations of the terms made above and below apply mutatis mutandis for all embodiments described in this specification and the accompanying claims (except it is stated that definition or explanation was given in the context of a specific method).

It has been found in the context of the present invention that the amount of sFlt1 in a sample of a subject suffering from ischemic stroke correlates to the extent of cardiac ischemia. Thus, by assessing changes of the amount of sFlt1 in a subject suffering from ischemic stroke it is possible to monitor the extent of cardiac ischemic.

Moreover, the present invention relates to a method for monitoring cardiac ischemia in a subject suffering from an ischemic stroke, said method comprising the steps of
a) determining the amount of sFlt1 (soluble fins-like tyrosine kinase-1) in a first sample from said subject
b) determining the amount of sFlt1 in a second sample from said subject obtained after said first sample, and
c) comparing the amount of sFlt1 in said first sample to the amount of sFlt1 in said second sample.

Preferably, said cardiac ischemia is monitored by carrying out the further step of d) monitoring said cardiac ischemia based on the result of the comparison carried out in step c). The subject shall preferably exhibit a preexisting heart disease at the onset of symptoms of said ischemic stroke. It has been shown in the context of the studies underlying the present invention that subject with a preexisting heart disease are prone to suffer from cardiac ischemia. A preexisting heart disease, preferably, is heart failure (systolic or diastolic heart failure). Further preferred preexisting heart diseases are dilatative and hypertrophic cardiomyopathy.

In general, there were no changes of the sFlt1 level after stroke. In some subjects, however, significant changes of the amount of sFlt1 were observed (see Examples). Some patients showed increases of sFlt1 after stroke. In other patients, the sFlt1 was decreasing after the onset of symptoms of stroke. The decreases and increases observed in the context of the present invention correspond to patients with an acute coronary syndrome (without a stroke). In patients with acute coronary syndromes, the sFlt1 increases shortly after the clinical event and decreases thereafter within hours. As found in acute coronary syndromes, the amount of sFlT1 rapidly increases after the clinical event and thereafter decreases within hours clearly indicating the presence of cardiac ischemia (see Examples). Accordingly, the term "monitoring" as used herein, preferably, refers to assessing cardiac ischemia in a subject suffering from ischemic stroke, and in particular to assess the extent of cardiac ischemia. More preferably, the term refers to assessing the course of cardiac ischemia in said subject, and in particular to assess the course of the extent of cardiac ischemia in said subject. Thus, by carrying out the aforementioned method, it can be particularly assessed whether cardiac ischemia has decreased, increased or remained unchanged within the period between obtaining the first and the second sample.

The term "cardiac ischemia" has been described elsewhere herein (see above).

Preferably, cardiac ischemia has increased if the amount of cardiac tissue (in particular the amount of myocardial tissue affected by ischemia) has increased during the period between obtaining the first and the second sample. Preferably, cardiac ischemia has decreased if the amount of cardiac tissue (in particular the amount of myocardial tissue) affected by ischemia has decreased increased during the period between obtaining the first and the second sample. Preferably, cardiac ischemia remained unchanged if the amount of cardiac tissue (and in particular the amount of myocardial tissue) affected by ischemia remained unchanged during the period between obtaining the first and the second sample. Preferably, a tissue is affected by ischemia, if the amount of oxygen that is supplied to said tissue is not sufficient in order to cover the need of the cells comprised by said tissue, and, thus, to meet the rate of mitochondrial oxidation in said cells.

As set forth above, by monitoring cardiac ischemia in subject suffering from ischemic stroke, it can be determined whether the condition of a said subject with respect to said cardiac ischemia improved or deteriorated between obtained the first and the second sample. As will be understood by those skilled in the art, such an assessment is usually not intended to be correct for 100% of the subjects to be monitored. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The first sample is, preferably, obtained after the onset of symptoms of ischemic stroke. It is also preferred that the first sample is obtained not more than 12, 24, 48, or more preferably not more than 72 hours after the onset of said symptoms. More preferably, the first sample is obtained between 15 minutes and 24 hours, between 15 minutes and 12 hours, between 15 minutes and 6 hours, or, more preferably, between 1 and 6 hours after the onset of said symptoms. Particularly preferred timepoints are 15 minutes, 1 hour, 2 hours, 3 hours, 6 hours or 12 hours after the onset of said symptoms.

The "second sample" is, preferably, understood as a sample which is obtained in order to reflect a change of the amount of the sFlt1 as compared to the amount of the respective marker in the first sample. The second sample shall be obtained after the first sample. Preferably, the second sample is not obtained too early after the first sample (in order to observe a sufficiently significant change to allow for monitoring). Preferably, the second sample is obtained 1 hour or not more than 1 hour, two hours or not more 2 hours, three hours or not more than 3 hours, 6 hours or not more than 6 hours, 9 hours or not more than 9 hours, 12 hours or not more than 12 hours after said first sample was obtained. Accordingly, it is particularly contemplated to obtain said second sample 1 to 12 hours, 2 to 9 hours, and, more preferably,3 to 6 hours after said first sample.

Preferably, at least one further sample is obtained in order to further monitor the change of the amount of sFlt1 to herein. Such further sample may be obtained, preferably, 1 to 12 hours, 2 to 9 hours, and, more preferably, 3 to 6 hours after said second sample.

The expressing "comparing the amount in said first sample as compared to the amount in said second sample" as used herein in the context of the encompasses comparing the amount of a sFlt1 in a first sample with an amount of said marker in a second sample. The terms "first sample" and "second sample" are specified herein above. It is to be understood that comparing as used herein refers to a comparison of corresponding parameters or values. It is to be understood that an amount of a marker is a first sample is compared to an amount of the amount the same marker, in a second sample. The comparison referred to in the context of the method of the present invention may be carried out manually or computer assisted. For a computer assisted comparison, the value of the determined amount in the second sample may be compared to a value of the determined amount in a first sample which is stored in a database by a computer program. The computer program may further evaluate the result of the comparison, i.e. automatically provide the desired assessment in a suitable output format. Based on the comparison of the amount of a biomarker as referred to herein in a first sample to the amount in a second sample it is possible to monitor cardiac ischemia in subject suffering from ischemic stroke.

As set forth above, the monitoring is, preferably, based on the comparison of the amount of a sFlt1 in a first sample to the amount of the sFlt1 in a second sample obtained after said first sample.

Preferably, an increased amount and, more preferably, a significantly increased amount, of sFlt1 in the second sample as compared to the amount of sFlt1 in said first sample is indicative for an increase of cardiac ischemia. Preferably, a decreased amount, and, more preferably, a significantly decreased amount of sFlt1 in said second sample as compared to said first sample is indicative for a decrease of cardiac ischemia. Preferably, an unchanged amount, and, more preferably, a significantly unchanged amount of sFlt1 in said second sample as compared to said first sample indicated unchanged cardiac ischemia and, thus, no change of the status of cardiac ischemia. Of course, said increased, decreased or unchanged status of cardiac ischemia is drawn to the period between obtaining the first and the second sample.

Particularly, a significant increase is an increase of a size which is considered to be significant, particularly said increase is considered statistically significant.

Particularly, a significant decrease is a decrease of a size which is considered to be significant, particularly said decrease is considered statistically significant.

The terms "significant" and "statistically significant" are known by the person skilled in the art. Thus, whether an increase or a decrease is significant or statistically significant can be determined without further ado by the person skilled in the art using various well known statistic evaluation tools which are also described elsewhere herein.

Moreover, an increase of the amount in the second sample as compared to the amount in the first sample which is considered to be significant is an increase of the amount in the second sample as compared to the first sample in a control subject or a group of control subjects in which cardiac ischemia has increased. Moreover, a decrease of the amount in the second sample of the biomarker as referred to herein as compared to the amount in the first sample which is considered to be significant is a decrease of the amount in the second sample as compared to the first sample in a control subject or a group of control subjects in which cardiac ischemia has decreased. Preferably, the period of time between obtaining the first and the second sample as well as the time points for obtaining said samples in the test subject corresponds to the period of time between obtaining the first and the second sample and to the time points for obtaining said samples in the control subject(s). Of course, the control subject shall also suffer from ischexnic stroke.

Preferred significant increases or decreases of the amount of sFlt1 which have been found in the course of the invention which indicate that cardiac ischemia has increased or decreased are given herein below:
According to the invention, an increase of the amount of sFlt1 in the second sample compared to the amount in the first sample, preferably, of at least 10%, more preferably of at least 20 %, more preferably of at least 30 %, more preferably of at least 40 %, even more preferably, of at least 50 %" more preferably of at least 60 %, more preferably of at least 70 %, more preferably of at least 80 %, more preferably of at least 90%, more preferably of at least 100%, more preferably of at least 150 % and most preferably of at least 200% is considered to be significant and, thus, to be indicative for an increase of cardiac ischemia.

Moreover, an increase of the amount of sFlt1 in the second sample compared to the amount in the first sample, preferably, of at least 5 pg/ml, more preferably of at least 10 pg/ml and even, more preferably, of at least 20 pg/ml or 30 pg/ml, and most preferably of at least 100 or 200 pg/ml is considered to be significant and, thus, to be indicative for an increase of cardiac ischemia.

According to the invention, a decrease of the amount of sFlt1 in the second sample compared to the amount in the first sample, preferably, of at least 10%, more preferably of at least 20 %, more preferably of at least 30 %, more preferably of at least 40 %, even more preferably, of at least 50 %" more preferably of at least 60 %, more preferably of at least 70 %, more preferably of at least 80 %, more preferably of at least 90%, more preferably of at least 100%, more preferably of at least 150 % and most preferably of at least 200% is considered to be significant and, thus, to be indicative for a decrease of cardiac ischemia. Moreover, a decrease of the amount of sFlt1 in the second sample compared to the amount in the first sample, preferably, of at least 5 pg/ml, more preferably of at least 10 pg/ml and even, more preferably, of at least 20 pg/ml or 30 pg/ml, and most preferably of at least 100 or 200 pg/ml is considered to be significant and, thus, to be indicative for a decrease of cardiac ischemia.

Advantageously, it has been found in the studies underlying the present invention that i) the determination of the amount of sFlt1 in a first sample and in a second sample of a subject suffering from ischemic stroke and ii) the comparison of the amount of sFlt1 in said first sample to the amount in said second sample allows for reliably monitoring cardiac. It was found that an increase of sFlt1 in a second sample as compared to a first (earlier obtained) sample indicates that cardiac ischemia has increased, whereas a decrease of sFlt1 in a second sample as compared to a first (earlier obtained) sample indicates that cardiac ischemia has decreased.

The assessment of cardiac ischemia is important, since it allows for monitoring the cardiac condition of the subject suffering from ischemic stroke. The aforementioned method allows for an early identification of a subject in which cardiac ischemia increases after the onset of symptoms of stroke. Said cardiac ischemia may be caused by the ischemic stroke of by a therapy for lowering the blood pressure initiated after the onset of symptoms of stroke (see elsewhere herein). The early identification of subjects in which cardiac ischemia increases after the onset of symptoms of stroke and/or after said therapy for lowering the blood pressure since it allows for further therapeutic measures which may prevent a further deterioration of the condition of the subject. Thanks to the present invention, such measures can be initiated before irreversible damage of cardiac tissue such as necrosis occurs.

Thus, the method of the present invention preferably also allows for diagnosing cardiac ischemia which is asymptomatic or clinically not apparent. Therefore, the invention also allows for diagnosing myocardial ischemia and, therefore, to assess a cardiac complication already before it becomes symptomatic or clinically apparent. Examples for cardiac complications which are typically not clinically apparent or asymptomatic are minor necrosis, and/or microinfarction. Thus, present invention is particularly useful in the diagnosis of minor ischemia, minor necrosis, and/or microinfarction. The term "clinically apparent" in this context particularly means that the coronary heart disease does cause a change in the shape of the electrocardiogram.

As set forth above, the invention allows to diagnose a cardiac ischemia before necrosis occurs. Consequently, the present invention also relates to determining a risk of suffering from a cardiovascular event such as unstable angina, STEMI or NSTEMI myocardial infarction caused by an ischemic stroke and/or by a therapy for lowering the blood pressure initiated after the onset of symptoms of stroke.

As laid out elsewhere herein, cardiac ischemia may be caused by a therapy for lowering the blood pressure. In this case the therapy for lowering the blood pressure shall be stopped in order to avoid further cardiovascular events.

Accordingly, the present invention also relates to method for identifying a subject being susceptible to a further therapy for lowering the blood pressure, said subject suffering from an ischemic stroke, said method comprising the steps of
a) determining the amount of sFlt1 (soluble fins-like tyrosine kinase-1) in a first sample from said subject
b) determining the amount of sFlt1 in a second sample from said subject obtained after said first sample,
c) comparing the amount of sFlt1 in said first sample to the amount in said second sample and
d) identifying a subject being susceptible to a further therapy for lowering the blood pressure based on the comparison carried out in step c.

Thus, the method of the present invention also allows for monitoring a therapy against ischemic stroke and, thus, supports the management of subject suffering from ischemic stroke.

Preferably, a subject being susceptible to a further therapy for lowering the blood pressure is identified based on the result of the comparison carried out in step c).

As described herein elsewhere, the subject shall, preferably, exhibit a preexisting heart disease at the onset of symptoms of said ischemic stroke.

The phrase "identifying a subject being susceptible to a further therapy for lowering the blood pressure" as used herein means assessing whether a subject suffering from an ischemic stroke who receives a therapy for lowering the blood pressure will be susceptible (and, thus, eligible) to a continuation of said therapy or not. A subject who is susceptible to a continuation of the said therapy, preferably, is not at risk of suffering from adverse side effects of said therapy if said therapy is continued, whereas a subject who is not susceptible to a continuation of the said therapy will be at risk of suffering from adverse side effects of the said therapy if said therapy. Adverse side effects of a therapy for lowering the blood pressure are, preferably, minor necrosis, microinfarction, unstable angina pectoris, Non-ST Segment Elevation myocardial infarction (NSTEMI), and ST Segment Elevation myocardial infarction (STEMI).

As will be understood by those skilled in the art, the assessment whether a subject is susceptible to a further therapy of lowering the blood pressure is usually not intended to be correct for all (i.e. 100%) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann- Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention.

The therapy for lowering the blood pressure may have been initiated before (e.g. if the patient exhibits a preexisting heart disease at the onset of symptoms of ischemic stroke) or after the onset of stroke symptoms.

In case that the therapy for lowering the blood pressure has been initiated after the onset of symptoms of ischemic stroke (e.g. shortly after presenting at the emergency room), said therapy, preferably, shall have been initiated within one to 48 hours, within one to 24 hours, within one to twelve hours, or within one to six hours, or, more preferably, within one to three hours after the onset of symptoms of ischemic stroke.

Therapies for lowering the blood pressure in patients with ischemic stroke are well known in the art. Particularly preferred therapies lowering the blood pressure are, e.g. disclosed by Adams et al. (loc. cit.). Preferably, the therapy for lowering the blood pressure shall lower arterial blood pressure. Also preferably, the therapy for lowering the blood pressure is selected from the group consisting of administration of calcium channel blockers, administration of beta andrenergic blockers, administration of angiotensin converting enzymes, administration of diuretics, administration of ACE (angiotensin converting enzyme) inhibitors and administration of renin inhibitors, vasodilators, nitroglycerin, alpha adenergic antagonists.

ACE-inhibitors are known to the person skilled in the art. Examples include benazepril, captopril, cilazapril, enalapril, fosinopril, lisinopril, moexipril, perindopril, quinapril, ramipril, spirapril, and trandolapril.

Beta adrenergic blockers (non-selective and βi -selective) are known to the person skilled in the art. Examples include acebutolol, alprenolol, atenolol, betaxolol, bisoprolol, bupranolol, carazolol, carteolol, carvedilol, celiprolol, metipranolol, metoprolol, nadolol, nebivolol, oxprenolol, penbutolol, pindolol, propanolol, sotalol, tanilolol, and timolol.

Preferred calcium channel blockers are Amlodipine (Norvasc), Aranidipine (Sapresta), Azelnidipine (Calblock), Barnidipine (HypoCa), Benidipine (Coniel), Cilnidipine (Atelec, Cinalong, Siscard), Clevidipine (Cleviprex), Isradipine (DynaCirc, Prescal), Efonidipine (Landel), Felodipine (Plendil), Lacidipine (Motens, Lacipil), Lercanidipine (Zanidip) Manidipine (Calslot, Madipine), Nicardipine (Cardene, Carden SR), Nifedipine (Procardia, Adalat), Nilvadipine (Nivadil), Nimodipine (Nimotop), Nisoldipine (Baymycard, Sular, Syscor), Nitrendipine (Cardif, Nitrepin, Baylotensin), Pranidipine (Acalas), and Verapamil.

Preferred diurectics are selected from the group consisting of thiazides (such a hydrochlorothiazide or bendroflumethiazide), loop diuretics (such as furosemide or bumetanide), and arginine vasopression receptor 2 antagonists.

Preferred vasodilators are nitrates selected from the group consisting of pentaerythrityl tetra-, tri-, di-and mononitrate, isosorbide mononitrate, isosorbide dinitrate and glycerol trinitrate.Particular preference is given to nitrates selected from the group consisting of pentaerythrityl tetranitrate, isosorbide mononitrate, isosorbide dinitrate, and glycerol trinitrate.

Preferred alpha adrenergic antagonists are prazosin, doxazoin, terazosin and phenoxybenzamine.

The first sample in the context of the aforementioned is, preferably, obtained after the onset of symptoms of ischemic stroke. Moreover, it is envisaged that said sample may be obtained before the therapy for lowering the blood pressure is initiated, preferably, within one hour before said therapy is initiated (in case said therapy is initiated after the onset of symptoms of ischemic stroke). It is also contemplated that the first sample is obtained not more than 1, 3, 6, or 9 hours after said therapy is initiated.

The "second sample" is, preferably, understood as a sample which is obtained in order to reflect a change of the amount of the sFlt1 as compared to the amount of the said marker in the first sample. The second sample shall be obtained after the first sample. In case the therapy for lowering the blood pressure was initiated after the onset of symptoms of stroke, the second sample shall be, preferably, obtained after the therapy for lowering the blood pressure was initiated. Preferably, the second sample is not obtained too early after the first sample (in order to observe a sufficiently significant change to allow for monitoring). Preferably, the second sample is obtained, two hours or not more 2 hours, three hours or not more than 3 hours, 6 hours or not more than 6 hours, 9 hours or not more than 9 hours, 12 hours or not more than 12 hours after said first sample was obtained. Accordingly, it is particularly contemplated to obtain said second sample between 1 to 12 hours, 2 to 9 hours, and, more preferably, between 3 to 6 hours after said first sample.

Preferably, an increase of the amount of sFlt1 in the second sample as compared to the first sample indicates that the subject is not susceptible to a further therapy of lowering the blood pressure. Preferably, said increase is a significant increase. Preferably, a decrease of the amount of sFlt1 or an essentially unchanged amount in the second sample as compared to the first sample indicates that the subject is susceptible to a further therapy of lowering the blood pressure. Preferably, said decrease is a significant decrease.

Particularly, a significant increase is an increase of a size which is considered to be significant, particularly said increase is considered statistically significant. The terms "significant" and "statistically significant" are known by the person skilled in the art. Thus, whether an increase is significant or statistically significant can be determined without further ado by the person skilled in the art using various well known statistic evaluation tools which are also described elsewhere herein.

Moreover, an increase of the amount in the second sample as compared to the amount in the first sample which is considered to be significant is an increase of the amount in the second sample as compared to the first sample in a control subject or a group of control subjects which are known not to be susceptible to a further therapy for lowering the blood pressure. Moreover, a decrease of the amount in the second sample of the biomarker as referred to herein as compared to the amount in the first sample which is considered to be significant is a decrease of the amount in the second sample as compared to the first sample in a control subject or a group of control subjects which are known to be susceptible to a therapy for lowering the blood pressure. Preferably, the period of time between obtaining the first and the second sample as well as the time points for obtaining said samples in the test subject corresponds to the period of time between obtaining the first and the second sample and to the time points for obtaining said samples in the control subject(s).

Preferred significant increases or decreases of the amount of sFlt1 which have been found in the course of the invention which indicate that cardiac ischemia are given herein below:
According to the invention, an increase of the amount of sFlt1 in the second sample compared to the amount in the first sample, preferably, of at least 40%, more preferably of at least 70 % and, even more preferably, of at least 100 %, and most preferably of at least 200% is considered to be significant and, thus, to be indicative for a subject not being susceptible to a further therapy for lowering the blood pressure.

Moreover, an increase of the amount of sFlt1 in the second sample compared to the amount in the first sample, preferably, of at least 40 pg/ml, more preferably of at least 70 pg/ml and even, more preferably, of at least 100 pg/ml or 150 pg/ml, and most preferably of at least 200 or 300 pg/ml is considered to be significant and, thus, to be indicative for a subject not being susceptible to a further therapy for lowering the blood pressure.

According to the invention, a decrease of the amount of sFlt1 in the second sample compared to the amount in the first sample, preferably, of at least 10%, more preferably of at least 20 %, more preferably of at least 30 %, more preferably of at least 40 %, even more preferably, of at least 50 %" more preferably of at least 60 %, more preferably of at least 70 %, more preferably of at least 80 %, more preferably of at least 90%, more preferably of at least 100%, more preferably of at least 150 % and most preferably of at least 200% is considered to be significant and, thus, to be indicative for a subject being susceptible to a further therapy for lowering the blood pressure. Of course, unchanged amounts of sFl1 are also indicative for a subject being susceptible to a further therapy for lowering the blood pressure. Moreover, increases of less than 5%, 10% or 15% are also indicative for a subject being susceptible to a further therapy for lowering the blood pressure.

Moreover, a decrease of the amount of sFlt1 in the second sample compared to the amount in the first sample, preferably, of at least 5 pg/ml, more preferably of at least 10 pg/ml and even, more preferably, of at least 20 pg/ml or 30 pg/ml, and most preferably of at least 100 or 200 pg/ml is considered to be significant and, thus, to be indicative for a subject being susceptible to a further therapy for lowering the blood pressure. Moreover, increases of less than 5 pg/ml, 10 pg/ml or 15 pg/ml are also indicative for a subject being susceptible to a further therapy for lowering the blood pressure.

Advantageously, it has been found in the studies underlying the present invention that i) the determination of the amount of sFlt1 in a first sample and in a second sample of a subject suffering from ischemic stroke and ii) the comparison of the amount of sFlt1 in said first sample to the amount in said second sample allows for assessing whether a therapy for lowering the blood pressure in a subject with ischemic stroke puts said subject at risk of certain adverse side effects (as described elsewhere herein). Therefore, by carrying out the aforementioned method, it can be assessed whether said subject is susceptible or not susceptible to a further therapy of lowering the blood pressure. Thanks to the present invention, adverse side effects of a treatment regimen that aims to lower the blood pressure in a subject suffering from ischemic stroke can be avoided.

Further, the present invention relates to the use of the biomarker sFlt1, or of a detection agent which specifically binds thereto in a sample of a subject suffering from an ischemic stroke for diagnosing cardiac ischemia associated with ischemic stroke in said subject.

The present invention also relates to the use of the biomarker sFlt1, or of a detection agent which specifically binds thereto in a sample of a subject suffering from an ischemic stroke for monitoring cardiac ischemia.

Moreover, the present invention relates to the use of the biomarker sFlt1, or of a detection agent which specifically binds thereto in a sample of a subject suffering from an ischemic stroke for identifying a subject being susceptible to a further therapy for lowering the blood pressure.

The term "detection agent" as used herein refers to an agent which is capable of specifically recognizing and binding to one of the biomarkers referred to above when present in a sample. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamers which specifically binds to the biomarker.

The present invention also pertains to a device adapted for carrying out the method of the present invention for diagnosing cardiac ischemia associated with ischemic stroke in a subject suffering from ischemic stroke, comprising
a) an analyzing unit comprising a detection agent which specifically binds to sFlt1, said unit being adapted for determining the amount of sFlt-1 in a sample of a said subject; and
b) an evaluation unit for comparing the determined amounts with a reference amount(s) whereby it can be diagnosed whether cardiac ischemia is the cause for said ischemic stroke, said unit comprising a database with reference amount values (a reference amount value) derived from a subject (or group of subjects) as defined above and a computer-implemented algorithm for carrying out a comparison as specified above.

The present invention also envisages a device adapted for carrying out the method of the present invention for monitoring cardiac ischemia in a subject suffering from ischemic stroke, comprising
a) an analyzing unit comprising a detection agent which specifically binds to sFlt1, said unit being adapted for determining the amount of sFlt-1 in a first and a second sample of a said subject; and
b) an evaluation unit for comparing the determined amount in the first and sample with the amount in the second sample whereby cardiac ischemia can be monitored, said unit comprising a database with values (a value) for increases (an increase) and/or decreases (an decrease) of the amount of sFlt1 in the second sample as compared to the first sample as defined above and a computer-implemented algorithm for carrying out a comparison as specified above.

Said increases, preferably, shall indicate that the cardiac ischemia has increased in said subject. Said decreases shall indicate that the cardiac ischemia has decreased in said subject. Preferred increases of the of the amount of sFlt1 in the second sample as compared to the first sample being indicative for an increase of cardiac ischemia and decreases of the of the amount of sFlt1. in the second sample as compared to the first sample being indicative for a decrease of cardiac ischemia are described elsewhere herein.

The present invention also envisages a device adapted for carrying out the method of the present invention for identifying a subject being susceptible to a therapy for lowering the blood pressure, comprising
a) an analyzing unit comprising a detection agent which specifically binds to sFlt1, said unit being adapted for determining the amount of sFlt-1 in a first and a second sample of a said subject; and
b) an evaluation unit for comparing the determined amount in the first and sample with the amount in the second sample whereby a subject being susceptible to a therapy for lowering the blood pressure can be identified, said unit comprising a database with values (a value for) increases (an increase) and/or decreases (an decrease) of the amount of sFlt1 in the second sample as compared to the first sample as defined above and a computer-implemented algorithm for carrying out a comparison as specified above.

Said increases shall, preferalbly, indicate that the subject is not susceptible to a therapy for lowering the blood pressure. Said decreases shall indicate that the subject is susceptible to a therapy for lowering the blood pressure. Preferred increases of the of the amount of sFlt1 in the second sample as compared to the first sample which indicate that the subject is not susceptible to a therapy for lowering the blood pressure, and preferred decreases of the of the amount of sFlt1 in the second sample as compared to the first sample which indicate that the subject is susceptible to a therapy for lowering the blood pressure are described elsewhere herein.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

The present invention contemplates a kit adapted for carrying out the method of the present invention for diagnosing cardiac ischemia associated with ischemic stroke in a subject suffering from ischemic stroke, said kit comprising a detection agent for the biomarker sFlt-1 as well as instructions for carrying out the said method.

The present invention contemplates a kit adapted for carrying out the method of the present invention for identifying a subject being susceptible to a therapy for lowering the blood pressure, said kit comprising a detection agent for the biomarker sFlt-1 as well as instructions for carrying out the said method.

The present invention contemplates a kit adapted for carrying out the method of the present invention for monitoring cardiac ischemia in a subject suffering from ischemic stroke, said kit comprising a detection agent for the biomarker sFlt-1 as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standards for reference amounts as described elsewhere herein in detail.

All references cited in this specification are herewith incorporated by reference with respect to their entire disclosure content and the disclosure content specifically mentioned in this specification.

The invention will be illustrated by the following Examples which, however, shall not be construed as limiting the scope.

### Example 1: Patients and determination of biomarkers

The biomarkers sFlt1, NT-proBNP and Troponin T were determined in serum samples in the following patient cohorts were investigated.

149 Persons, mean age 42.8 years were tested as apparently healthy controls, they were symptomatic, had no history of cardiac risk factors and a normal kidney function as assessed by creatinine.

42 patients with stable heart failure and established CAD were used as a second control group, they were on stable medication, nor allowed to change weight of more than 2 kg within 3 months and had normal kidney function.

254 patients with stroke (mean age 70 years) were also tested. Ischemic stroke was confirmed by imaging (MRI scan or CT scan), stroke was separated into TIA (transitory ischemic attack), minor and major stroke according to the size of the stroke by imaging. Samples were obtained at presentation (between 2.5 and 34 hours after the onset of stroke symptoms, Median: 7.3 h) as well as 6 hours and 24 hours after presentation.

The following methods and devices were used for determining the amount of the biomarkers referred to herein:
sFlt1, Troponin T (hsTNT), and NT-proBNP were determined with sandwich immunoassays using analyzers from Elecsys or COBAS e-series. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. sFlt-1 amounts between 10 to 85,000 pg/ml, GDF-15 amounts between 300 pg to 20,000 pg/ml, and NT-proBNP amounts between 2 pg/ml and 35,000 pg/ml can be measured. The high sensitivity Troponin T test used in this study has a sensitivity of 2 pg/ml. The amounts of sFlt1, Troponin T and NT-proBNP in Examples are given in "pg/ml".

### Example 2: sFlt1 in apparently healthy subjects patients, in patients with stable heart failure and established coronary artery disease (HF/CAD) and patients with ischemic stroke

Table 1 shows the sFlt1 levels in patients in the three tested patient cohorts. The 25^{th}, 50^{th} and 75^{th} percentiles are indicated (pg/ml).

**Table 1**

| sFltl pg/ml | normal subjects | HF/CAD | Stroke |
|---|---|---|---|
| 25 % | 42 | 49 | 52 |
| | | | |
| 50 % | 52 | 74 | 60 |
| | | | |
| 75 % | 71 | 81 | 74 |

The table clearly indicates that sFlT1 levels were in average not elevated in patients suffering from stroke relative to patients suffering patients from stable heart failure and established coronary artery disease (HF/CAD) or relative to healthy control patients. In order to further dissect the patient group with stroke sFlT1 levels were correlated to size of stroke and to time from event to time of blood sampling. This is surprising since sFlt1 has been proposed as marker for stroke (see e.g. US2007/218498)

### Example 3: sFlt1 in different stroke categories

Table 2 shows the Flt-1 in patients with transient ischemic attack (TIA), with minor and major stroke (again the 25^{th}, 50^{th} and 75^{th} percentile is indicated)

**Table 2**

| sF1Tl | TIA | minor Stroke | major stroke |
|---|---|---|---|
| | n=79 | n=61 | n=104 |
| 25 % | 51 | 51 | 54 |
| | | | |
| 50 % | 56 | 61 | 62 |
| | | | |
| 75 % | 65 | 75 | 76 |

Interestingly, the sFlt1 levels did not significantly correlate with stroke size and are not different from control groups.

### Example 3: sFlt1 at various timepoints with respect to the onset of stroke symptoms

Samples were obtained less than 6 hours after the onset of stroke symptoms, within a period of 6 to 12 hours after the onset of stroke symptoms, and more than 12 hours after the onset of stroke symptoms. The sFlt1 levels in these samples are given in table 3 (in pg/ml).

**Table 3: sFlT1 levels in comparison from time to event to time of blood sampling:**

| | Below 6 h | 6-12 h | above 12 h |
|---|---|---|---|
| | | | |
| TIA | 56 | 57 | 66 |
| N=79 | (51-65) | (50-69) | (59-89) |
| | | | |
| | | | |
| Minor Stroke | 61 | 57 | 67 |
| N=61 | (52-75) | (51-69) | (57-83) |
| | | | |
| Major stroke | 62 | 64 | 67 |
| N=104 | (54-76) | (51-106) | (53-90) |

The Table clearly illustrates that time from ischemic event to blood sampling and size of stroke does not significantly impact sFlTl levels indicating that cerebral ischemia is not associated with increased levels of sFlT1.

### Example 4: sFlt1 levels in patients with cardiac ischemia

Interestingly, significantly increased sFlt1 levels were found in samples obtained from three stroke patients at presentation. A patient (case 1) who presented 2.5 hours after onset of stroke symptoms, had a sFlt1-level of 120 pg/ml at admission, a further patient (case 2) had a sFlt1-level of 169 pg/ml (measured in a sample obtained 5 hours after the onset of stroke symptoms), another patient (case 3) had a sFlt1-level of 115 pg/ml. The levels observed in these patients were even larger than the 95^{th} percentile in HF/CAD patients. In patients 2 and 3, the sFlt1 sFlt1 levels had returned to normal after presentation.

| Case 1: | sFlT1 | TNT | NT-pro BNP |
|---|---|---|---|
| At presentation | 120 | 29 | 402 |
| | 136 | 21 | 1373 |
| | 111 | 24 | 1379 |
| | | | |
| Case 2 (at present.) | 115 | 0 | 238 |
| | 49 | 0 | 249 |
| | 50 | 0 | 463 |
| | | | |
| Case 3: (at present.) | 160 | 7 | 178 |
| | 49 | 5 | 120 |
| | 58 | 11 | 58 |

The samples were obtained at presentation as well as 6 and 24 hours after obtaining the first sample.

In patients 1 and 2, a troponin T increase was not observed, indicating that cardiac ischemia did not result in myocardial necrosis (which is, e.g. the case in patients with unstable angina pectoris). However, ECG abnormalities were found in patient 2 who showed a ST depression of up two 2 mm indicating the presence of ischemia. In patient 3, a slight increase of Troponin T was observed 24 hours after admission. The increased Troponin T levels indicate cardiac micronecrosis.

As a control, the amounts of sFlt1 were measured in two patients presenting with an ACS (acute coronary syndrome) at the emergency room. The patients did not suffer from ischemic stroke. At admission, the subjects had sFlt1 serum levels of 139 pg/ml and 298 pg/ml, respectively. Later it was shown that both subjects suffered from Non ST myocardial infarction. Thus, the amounts of sFlt1 in patients 1 to 3 correspond to the amounts of patients with ACS at admission.

As a further control, the amount of sFlt1 was determined in a patient with cardiac ischemia induced by stent implantation. Samples were obtained 1, 5 and 24 hours after stent implantation. The sFlt1 level in the 1 h samples was increased (106 pg/ml). In the 5 h sample, the amount was even further increased (374 pg/ml). 24 hours after stent implantation a level of 161 pg/ml was measured. Thus, the sFlt1 levels in the three patients described above correspond to levels observed in cardiac ischemia induced by stent implantation.

The data show that sFlT1 at presentation is a useful tool to identify individuals who have suffered from cardiac ischemia associated with ischemic stroke. Moreover, the data strongly indicated that it is posssible to assess whether cardiac ischemia is a trigger for stroke, i.e. the cause of stroke. As mentioned above, stroke was usually not associated with the induction of sFlT1 increase as found in a large number of patients with different severity of stroke and samples taken early and late. Among three patients with sFlT1 increases in two cases this could be clearly attributed to cardiac causes as shown by early ECG changes or subsequent troponin T increase, both being independent indicators of cardiac ischemia and the latter being a specific indicator of cardiac necrosis. These patients deserve more intense follow up with ECG and further cardiac work up to reduce the risk or severeness of cardiac damage (echocardiography and/or CT scan and angiography as needed).

### Example 4: Monitoring cardiac ischemia

At 6 h after presentation, 63 patients had increases of sFlT1 of more than 10 % indicating ischemic complications related to the underlying disease or therapy induced, 50 of these patients had a history of hypertension and 13 a history of diabetes mellitus, indicating that ischemia occurred exclusively in risk groups. In 37 of the patients with hypertension blood pressure was therapeutically reduced. Moreover, 43 patients had sFlt1 increases of more than 20% indicating cardiac ischemia. Patients showing increasing levels of sFlt1 (more than 10%) were classified as "risk patients" (since they show stroke induced cardiac ischemia). The other patients were classified as "non-risk patients". The patients had normal kidney function at presentation.

As an example the course of the sFlt1 levels is shown in patients 5 and 6 (stroke patients).

| Case 4: | |
|---|---|
| At presentation: | 58 pg/ml |
| 6 hours after presentation | 177 pg/ml |
| 24 hours after presentation | 86 pg/ml |
| | |

| Case 5 | |
|---|---|
| At presentation: | 81 pg/ml |
| 6 hours after presentation | 264 pg/ml |
| 24 hours after presentation | 425 pg/ml |

In order to further identify groups of increased risk of cardiac ischemia, baseline levels for NT-pro BNP, sensitive Troponin T and GDF 15 were determined at presentation. This is summarized ion the following Table (Risk Group: patients with a more than 10% increase of sFlt1, Non Risk Group: Remaining patients):

| | | | |
|---|---|---|---|
| | NT-pro BNP | sens Troponin T | GDF 15 |
| | pg/ml | pg/ml | pg/ml |
| Total Group | 268 | 6,8 | 1146 |
| N = 229 | (79-754) | (0,0-15,3) | (831-1911) |
| | | | |
| Risk Group | 497 | 8,7 | 1405 |
| N = 63 | (127-1185) | (0,0-17,3) | (946-2216) |
| | | | |
| Non-Risk Group | 211 | 5,8 | 1131 |
| N=164 | (76-632) | (0,0-14,4) | (818-1595) |

Interestingly, patients with increased NT-proBNP, Troponin T and GDF-15 levels belong to the group of patients with an increase of sFlt1 of more than 10% (risk group). These patient most likely exhibited a pre-existing heart disease such as heart failure at the onset of symptoms if stroke. Since a larger number of these patients showed an sFlt1 increase, they need more intense monitoring including sFlt1 (since they are at increased risk of ischemia).

In order to assess the impact of sFlT1 increases median values of the total group of patients and the risk groups were compared in terms of increase in NT-pro BNP, sens Troponin T and GDF 15. This is shown in the following Table

| | | Total Group | Risk Group | Non-Risk Group |
|---|---|---|---|---|
| | | | | |
| NT-pro BNP | baseline | 268 | 497 | 211 |
| | 6 h | 286 | 730 | 248 |
| | 24 h | 333 | 413 | 270 |
| Sens Trop | T baseline | 6,8 | 8,7 | 5,8 |
| | 6 h | 7,1 | 9,1 | 6,5 |
| | 24 h | 7,9 | 9,0 | 7,3 |
| | | | | |
| GDF-15 | baseline | 1146 | 1405 | 1131 |
| | 6 h | 1185 | 1375 | 1153 |
| | 24 h | 1097 | 1161 | 1052 |

Risk patients differed from the control group by an increase in NT-pro BNP suggesting deterioration of cardiac disorder in this risk population. In generally, troponin T was not increased in the risk group. However, in some patients with a significant sFlt1 increase, an increase of Troponin T was observed (see e.g. case 6) which indicated micronecrosis. In some cases with a signir

Case 6: A first sample (serum) was obtained at presentation (11 hours after the onset of stroke symptoms). Two further serum samples were obtained 6 and 24 hours after presentation. The amounts of sFlt1 were measured in these samples (see table 3). The patient had a significant increase of sFlt1 followed by an increase of Troponin T. In this case, cardiac ischemia resulted in micronecrosis (as indicated by the increase of Troponin T (which indicates cardiac ischemia followed by micronecrosis).

| Case 6 | sFlT1 | TNT | Nt-pro BNP |
|---|---|---|---|
| | 66 | 8 | 1547 |
| | 645 | 7 | 1478 |
| | 503 | 11 | 4076 |

### Example 5

A 67 years old patient is admitted to the emergency room with right sided hemiparesis, which occurred 4 hours ago, he has a history of smoking and heart failure and takes diuretics, ACE inhibitors and beta blockers on a regular basis to keep his blood pressure low. At admission his blood pressure is 110/65 mmHg. His NT-pro BNP in 630 pg/ml, troponin T is 8 pg/ml and sFlT1 is 81 pg/ml, 6 hours later his sFlT1 increases to 261 pg/ml, troponin T remains unchanged and NT-pro BNP has increased to 815 pg/ml, 2 hours before his blood pressure has dropped to 95/60 mmHg. Heart failure drugs are discontinued and sFlT1 returns as well as the blood pressure (which is 115/75 mmHg 4 hours later) returns to normal. The patient's symptoms are slightly improved.

The case shows that a significant sFlt1 increase indicating cardiac ischemia can be observed in stroke patient treated with blood pressure lowering drugs. The increase of sFlt1 is accompanied by significant drop of blood pressure. After the treatment with blood pressure lowering medication is stopped, the sFlt1 levels as well as the blood pressure return to normal. Thus, based on the determination of the amount of sFlt1 in a patient suffering from stroke, decisions can be made whether a therapy for lowering the blood pressure shall be continued or discontinued.

### Example 6

A 64 year old patient experienced severe headache 12 h ago followed by left sided weakness of his arm. At presentation at the emergency room his blood pressure is 240/140 mm Hg and a hypertensive crisis is diagnosed. A serum sample is obtained at presentation (NT-pro BNP: 215 pg/ml, Troponin T: 4 pg/ml and sFlT1:76 pg/ml). His blood pressure is immediately treated with clonidine, an antihypertensive drug, and drops to 145/100 mmHg. 6 hours later sFlT1 increased to 185 pg/ml while NT-pro BNP and troponin T remained unchanged. Symptoms of stroke have worsened in between (weakness of the left; arm intensified however headache improved). After treatment adjustment, blood pressure increases to 170/100 mg and weakness of the left arm improves, indicating improvement of stroke symptoms likely to be associated with improved perfusion. Moreover, sFlT1 normalises. The case shows that sFlt1 allows for making decisions whether a therapy with a blood pressure lowering drug shall be continued or not.

### Example 7

A 73 year old patient presents with dysarthria lasting 6 h at presentation to the emergency room, sFlT1 is normal (76 pg/ml) and also on follow-up at 6 and 24 h. NT-pro BNP is continuously recorded at 160 pg/ml and troponinT at 3 pg/ml throughout the observation period. Symptoms gradually improve during the observation time. Thus, not changes of sFlt1 are observed indicated that the patient did not exhibit cardiac ischemia.

### Example 8

A 56 years old patient with a history of coronary artery disease became right sided hemiparetic. Therefore, he presents to the emergency room four hours after the onset of stroke symptoms. He is unable to respond to questions, his sFlT1 is 185 pg/ml, NT-pro BNP is 260 pg/ml and troponinT is 6 pg/ml (in a sample obtained at presentation). His ECG shows ST depression compatible with cardiac ischemia, blood pressure was 140 /90 mm Hg. On follow up sFlT1 normalised and troponin T slightly increased to 11 pg/ml indicating necrosis. The patient is diagnosed with cardiac ischemia followed by cardiac necrosis which induced ischemic stroke.

The case shows that the determination of sFlt1 in a sample of a subject allows for diagnosing cardiac ischemia associated with an ischemic stroke. In this case the cardiac ischemia is followed by micronecrosis (as indicated by the increased Troponin T level).

### Summary:

sFlt1 is not generally increased in ischemic stroke, let alone an indicator of the severity of stroke. However, increased sFlT1 levels at the onset of stroke symptoms (or shortly after the onset of symptoms of stroke) are an indication of ischemic heart disease as a potential cause of stroke. Increases of sFlT1 in the course of a heart disease indicate worsening of the heart disease preferably in those patients who already had advanced heart disease when stroke occurred.

Thus, sFlT1 is of value in detecting underlying causes of stroke as well as complications during stroke and therefore has the potential of directing appropriate diagnostic and therapeutic steps.

## Claims

1. A method for diagnosing cardiac ischemia accociated with ischemic stroke in a subject suffering from an ischemic stroke, said method comprising
a) determining the amount of soluble fins-like tyrosine kinase-1 (sFlt1) in a sample from said subject, and
b) comparing the amount of sFlt1 in said sample to a reference amount, whereby it is diagnosed whether cardiac ischemia is associated with ischemic stroke.

2. The method of claim 1, wherein said sample is obtained up to six hours after the onset of symptoms of ischemic stroke.

3. The method of claims 1 and 2, wherein the reference amount is derived from a subject or a group of subjects known to suffer from an ischemic stroke, wherein cardiac ischemia is associated with said ischemic stroke, wherein an essentially identical amount or an increased amount of sFlt1 in the test sample as compared to reference amount is indicative for cardiac ischemia is associated with the ischemic stroke.

4. The method of any one of claims 1 to 3, wherein the reference amount is derived from a subject or a group of subjects known to suffer from an ischemic stroke, wherein cardiac ischemia is not associated with said ischemic stroke, wherein an essentially identical amount or a decreased amount of sFlt1 in the test sample as compared to reference amount indicates that cardiac ischemia is not associated with said ischemic stroke.

5. A method for monitoring cardiac ischemia in a subject suffering from an ischemic stroke, said method comprising the steps of
a) determining the amount of sFlt1 in a first sample from said subject
b) determining the amount of sFlt1 in a second sample from said subject obtained after said first sample, and
c) comparing the amount of sFlt1 in said first sample to the amount of sFlt1 in said second sample.

6. The method of claim 5, wherein said first sample is obtained not more than one hour after the onset of symptoms of stroke, and/or wherein said second sample is obtained three to twenty-four hours after said first sample.

7. The method of claims 5 and 6, wherein an increased amount of sFlt1 in said second sample as compared to the amount of sFlt1 in said first sample is indicative for an increase of cardiac ischemia and/or wherein a decreased amount of sFlt1 in said second sample as compared to said first sample is indicative for a decrease of cardiac ischemia.

8. A method for identifying a subject being susceptible to a further therapy for lowering the blood pressure, said subject suffering from an ischemic stroke, said method comprising the steps of
a) determining the amount of sFlt1 (soluble fins-like tyrosine kinase-1) in a first sample from said subject
b) determining the amount of sFlt1 in a second sample from said subject obtained after said first sample,
c) comparing the amount of sFlt1 in said first sample to the amount in said second sample and
d) identifying a subject being susceptible to a further therapy for lowering the blood pressure based on the comparison carried out in step c.

9. The method of claim 10, wherein a decrease of the amount of sFlt1 in the second sample as compared to the first sample indicates that the subject is susceptible to a further therapy of lowering the blood pressure and/or wherein an increase of the amount of sFlt1 in the second sample as compared to the first sample indicates that the subject is not susceptible to a further therapy of lowering the blood pressure.

10. The method of any one of claims 1 to 9, wherein the amount of TGFβ-1 is determined instead of the amount of sFlt1.

11. Use of the biomarker sFlt1, or of a detection agent which specifically binds thereto in a sample of a subject suffering from an ischemic stroke for diagnosing cardiac ischemia associated with ischemic stroke in said subject.

12. Use of the biomarker sFlt1 or of a detection agent which specifically binds thereto in a sample of a subject suffering from an ischemic stroke for monitoring cardiac ischemia.

13. Use of the biomarker sFltl, or of a detection agent which specifically binds thereto in a sample of a subject suffering from an ischemic stroke for identifying a subject being susceptible to a further therapy for lowering the blood pressure.

14. A kit adapted for carrying out the method of claim 1 to 4, said kit comprising a detection agent for the biomarker sFlt-1 as well as instructions for carrying out the said method.

15. A device adapted for carrying out the method of the present invention for diagnosing cardiac ischemia associated with ischemic stroke in a subject suffering from ischemic stroke, comprising
a) an analyzing unit comprising a detection agent which specifically binds to sFltl, said unit being adapted for determining the amount of sFlt-1 in a sample of a said subject; and
b) an evaluation unit for comparing the determined amounts with a reference amount(s) whereby it can be diagnosed whether cardiac ischemia is the associated with said ischemic stroke, said unit comprising a database with a reference amount values (a reference amount value) derived from a subject or group of subjects as defined in claims 3 and/or 4, and a computer-implemented algorithm for carrying out a comparison as specified above.
